(19) Europäisches Patentamt / European Patent Office / Office européen des brevets

(11) **EP 4 648 734 B1**

(12) **EUROPEAN PATENT SPECIFICATION**

(45) Date of publication and mention
of the grant of the patent:
**18.02.2026 Bulletin 2026/08**

(21) Application number: **25717063.9**

(22) Date of filing: **04.04.2025**

(51) International Patent Classification (IPC):
*A61K 8/02* (2006.01)   *A61K 8/26* (2006.01)
*A61K 8/49* (2006.01)   *A61Q 17/04* (2006.01)
*A61K 8/25* (2006.01)   *A61K 8/35* (2006.01)
*A61K 8/37* (2006.01)

(52) Cooperative Patent Classification (CPC):
**A61Q 17/04; A61K 8/25; A61K 8/35; A61K 8/37;
A61K 8/4966;** A61K 2800/612; A61K 2800/652

(86) International application number:
**PCT/EP2025/059380**

(87) International publication number:
**WO 2025/210278 (09.10.2025 Gazette 2025/41)**

(54) **SUNSCREEN COMPOSITION WITH FUNCTIONALIZED ZEOLITE SPF BOOSTERS**

SONNENSCHUTZZUSAMMENSETZUNG MIT FUNKTIONALISIERTEN ZEOLITH-SPF-BOOSTERN

COMPOSITION D'ÉCRAN SOLAIRE À RENFORÇATEURS DU FACTEUR DE PROTECTION SOLAIRE À ZÉOLITES FONCTIONNALISÉES

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **05.04.2024  IT 202400007582
22.07.2024  IT 202400016930**

(43) Date of publication of application:
**19.11.2025  Bulletin 2025/47**

(73) Proprietor: **Saes Getters S.p.A.
20045 Lainate (MI) (IT)**

(72) Inventors:
• **RIVA, Miriam**
22074 Lomazzo (CO) (IT)
• **FIDECKA, Katarzyna**
22063 Cantù (CO) (IT)
• **DE SIMONE, Agnello**
21047 Saronno (VA) (IT)

(74) Representative: **Novagraaf Group
Chemin de l'Echo 3
1213 Onex / Geneva (CH)**

(56) References cited:
WO-A1-2018/140304    CA-A1- 3 032 422
CN-A- 109 395 768    KR-B1- 101 229 509
US-A1- 2005 276 761

• FANTINI RICCARDO ET AL: "Boosting sunscreen stability: New hybrid materials from UV filters encapsulation", MICROPOROUS AND MESOPOROUS MATERIALS, vol. 328, 2 October 2021 (2021-10-02), Amsterdam ,NL, pages 111478, XP055863073, ISSN: 1387-1811, DOI: 10.1016/j.micromeso.2021.111478
• MICHELLE N CHR�TIEN ET AL: "Reducing adverse effects from UV sunscreens by zeolite encapsulation: comparison of oxybenzone in solution and in zeolites", PHOTOCHEMISTRY AND PHOTOBIOLOGY, vol. 86, no. 1, 18 November 2009 (2009-11-18), US, pages 153 - 161, XP055459736, ISSN: 0031-8655, DOI: 10.1111/j.1751-1097.2009.00644.x

EP 4 648 734 B1

**Description**

**FIELD OF INVENTION**

The present invention relates generally to sunscreen compositions for protecting damage from ultraviolet (UV) rays. More specifically, the invention is direct to a sunscreen formulation with SPF boosters based on specific zeolites functionalized with a silane moiety.

**DESCRIPTION**

The negative effects of exposure to ultraviolet (UV) light having a wavelength of from about 200 nm to about 400 nm, are generally recognized. The prolonged non-shielded exposure to solar radiation causes adverse health consequences, such as the immediately appearing of painful sunburns, and long-term damage which can lead to serious conditions such as skin cancer.

UV light also contributes to aging by causing free radicals to form in the skin. Free radicals include, for example, singlet oxygen, hydroxyl radical, the superoxide anion, nitric oxide and hydrogen radicals. Free radicals attack DNA, membrane lipids and proteins, generating carbon radicals. These in turn react with oxygen to produce a peroxyl radical that can attack adjacent fatty acids to generate new carbon radicals. This cascade leads to a chain reaction producing lipid peroxidation products. Damage to the cell membrane results in loss of cell permeability, increased intercellular ionic concentration, and decreased ability to excrete or detoxify waste products.

In order to reduce the amount of solar UV radiation received by the skin during exposure to the sun radiation, different sunscreen compositions can be used.

A sun care composition may contain inorganic UV filters, also called physical filters, and/or chemical UV filters which are organic molecules. The inorganic UV filters interact with UV light by two mechanisms: absorption and reflection/scattering, while the organic filters typically contain aromatic carbon and/or other electron-dense bonds that are responsible for absorbing light in the UV ranges of the solar spectrum.

The higher the amount of UV filters, the greater the degree of UV protection, however, it has recently become evident that too high concentration of both inorganic and organic UV filters, not only compromise the sun care formulation aesthetics, but it also brings unfavorable safety impacts on both human health and the environment.

Therefore, a key challenge that arose in this field is represented by the reduction of the quantities of UV filters used in sunscreen formulations while still ensuring high sun protection efficacy.

According to that, an effective strategy to solve this problem involves the use of a sun protection factor (SPF) booster into sunscreen formulations. SPF boosters are defined as compounds, safe for both human use and the environment, which are not recognized as sunscreen actives, but work to increase the SPF of the composition in which they are incorporated.

Typical sunscreen formulations are emulsions, serums, creams, or gels containing, in addition to chemical and/or physical UV filters, many other compounds such as emulsifiers, solubilizers, stabilizers, preservatives and also SPF boosters; all of which influence the protectiveness of the sunscreen, the activity of the SPF Booster and the and cosmetic appeal.

As reported in WO2017112982 and WO2021102873 a widely used inorganic SPF booster is represented by hydrophobic silica particles, also in the form of silica particles whose surface is treated with silylating agents. US9265715 claims for example the use of silica particles in combination with nylon and barium sulfate particles in order to have a boosting effect on sunscreen composition. US2005276761 discloses sunscreen compositions comprising zeolites and sunscreen agents.

A further example is represented by US20210330571 in which hydrophobic fine particulate of titanium oxide or zinc oxide, ensure a boosting effect to the final composition. Alternatively, WO2022081942 describes an organic compound, specifically xanthommatin, as SPF booster in combination with a chemical UV filter.

However, some of the most cited classes of compounds such as silica has been progressively reduced due to their toxicity for human and environment. While inorganic compounds as titanium oxide and zinc oxide or organic compounds as the xanthommatin above reported, cannot be considered an SPF booster since they act as a real UV filter (the so-called SPF-doping effect).

Thus, object of the present invention is a sunscreen composition comprising at least one UV filter and at least a specific type of zeolite selected among Beta type zeolite, ZSM-5 type (MFI), Linde type (LTA), Mordenite type (MOR) or Faujasite type (FAU) functionalized with a silane moiety selected among thiethoxycaprylyl silane Stearyl Triethoxysilane, Trihydroxymethylsilane and Methoxypolyethyleneoxypropyltrymetoxysilane

In particular, the inventors found that said specific functionalized zeolites, which do not show a sunscreen activity as a single component, act as an efficient SPF booster when formulated in a sunscreen formulation comprising UV filters, thus avoiding the SPF doper effect according to the international safety regulation.

Moreover, it has been clearly pointed out that, the functionalization of said zeolites and the consequent amphiphilic nature acquired, improves the dispersibility of the same in both one-phase and two-phase formulations and reduces the aggregation phenomena in the final formulation with a consequent significant increase of the SPF Boosting effect. Indeed, as reported in the below experimental part, the boost ensured by said specific zeolites to the final composition SPF is at least 20%.

Said SPF boosters have also been proved to be compatible both with marine friendly UV filters and other marine friendly sunscreens' components, and with water resistant components for water resistant sunscreen formulations.

In a preferred embodiment, the zeolites are characterized by the presence of ammonium or hydrogen as counterion at an ion exchange site of the framework structure with a pH comprise in a range between 3.0 and 8.0.

In a further aspect, the zeolites are characterized by a particle size distribution is comprised between 0.1 and 15 $\mu$m, preferably between 0.2 to 5.0 $\mu$m.

The functionalization of the zeolites are made with a silane selected among thiethoxycaprylyl silane Stearyl Triethoxysilane, Trihydroxymethylsilane or Methoxypolyethyleneoxypropyltrymetoxysilane in an amount comprised between 0.2 and 10.0 %, preferably between 2.0 and 6.0 % with respect to the total weight of the zeolite.

According to the invention, the amount of zeolites is comprised between 0.1 and 5.0 %wt, preferably between 1.0 and 3.0%wt with respect to the total weight of the composition; while the amount of the UV filter is comprised between 1.0 and 35%wt with respect to the total weight of the composition.

In a preferred embodiment, specifically when an organic UV filter is employed, the amount of said compound is comprised between 1 and 15% wt with respect to the total amount of the composition, while the amount is preferably comprised between 1 and 35%wt with respect to the total amount of the composition, when said filter is an an inorganic compound.

Indeed, the UV filters employed in the sunscreen composition described herein can be an organic molecule, also defined as chemical filter, an inorganic compound, also defined as physical filter or mixture thereof.

Said organic molecules are selected in a group consisting of Octocrylene, Ethylhexyl Salicylate, Homosalate, Butyl Methoxydibenzoylmethane, Diethylamino hydroxybenzoyl hexyl benzoate, Bis-Ethylhexyloxyphenol Methoxyphenyl Triazine, Ethylhexyl Triazone, Avobenzone, Diethylhexyl Butamido Triazone, Ethylhexyl Methoxycinnamate, Phenylbenzimidazole sulfonic acid, Tris-biphenyl triazine, Tris-biphenyltrazine (nano), Oxybenzone, Octinoxate, Octyltriazone, Octisalate, Padimate O, Bis-piperazine HAA299, Bis-piperazine HAA299 (nano), (2-Ethoxyethyl (2Z)-2-cyano-2-[3-(3-methoxypropylamino) cyclohex-2-en-1-ylidene]acetate), Sulisobenzone, Drometrizole trisiloxane, Methylene bis-benzotriazolyl tetramethylbutylphenol, Benzylidene camphor sulfonic acid, 4-Methylbenzylidene Camphor, Polysilicone-15, Sulisobenzone sodium, Aminobenzoic acid, PEG-25 PABA, Methylene Bis-Benzotriazolyl Tetramethylbutylphenol, Methylene Bis-Benzotriazolyl Tetramethylbutylphenol nano, Menthyl anthranilate, Isopentyl-4-methoxycinnamate, terephthalylidene dicamphor sulfonic acid, Dioxibenzone, Cinoxate, camphor benzalkonium sulfate and mixtures thereof possibly considering all size ranges both nano-, sub- and micro-metric, including modification by any means (via chemical or physical approaches).

While the inorganic compounds are selected among Zinc Oxide, Anatase titanium dioxide, Rutile, titanium dioxide and mixtures thereof possibly considering all size ranges both nano-, sub- and micro-metric.

As reported above, the SPF boosters herein described are extremely versatile and compatible in several matrixes, thus according to the present invention, the final sunscreen composition can be formulated either as a single-phase formulation, such as a gel or a stick, or as a two-phase formulation such as an homogenous oil-in-water emulsion, such as a serum or a cream.

The sunscreen composition according to the invention can also comprise at least one of a cosmetically acceptable emollient, carrier fluid, pigment, humectant, vitamin, antioxidant, emulsifier, co-emulsifier, hydrophilic or hydrophobic thickeners, wax, butter, film former, silicone, surfactant, active agent, extract, fragrance, preservative, pH-adjusting agent, suspending agent, chelating agent.

The present invention also refers to the composition according to any of the embodiment herein disclosed as a sunscreen composition or agent. In particular, the invention refers to a composition according to any of the embodiment herein disclosed for use in a method for protecting a keratinous surface (e.g. skin) from UV radiation comprising contacting the keratinous surface with the sunscreen composition of the invention.

The invention also refers to the composition as herein disclosed as an additive or adjuvant to a sunscreen composition. In addition, the invention refers to the zeolites or functionalized zeolites as disclosed herein, particularly selected in a group consisting of Beta type, ZSM-5 type (MFI), Linde type (LTA), Mordenite type (MOR) and Faujasite type (FAU) functionalized with a silane moiety selected among thiethoxycaprylyl silane Stearyl Triethoxysilane, Trihydroxymethylsilane or Methoxypolyethyleneoxypropyltrymetoxysilane, for use as adjuvant in sunscreen compositions or for use in boosting the SPF effect of UV filters in sunscreen compositions.

Surprisingly, the SPF boosting effect is reached by the compositions and/or functionalized zeolites of the invention without the zeolite binding to the filter, e.g., with no absorption or encapsulation. According to the present invention, in fact, the zeolite and the UV filter do not interact but simply form a physical mixture. As demonstrated by SEM images (FESEM - EDS (Supra 55VP / Zeiss + UltraMax 170mm2 / Oxford Instruments)), according to the present invention, the zeolite is not integrated and is not part of the UV filter and does not act as a carrier. As will be apparent from the following experimental section, the zeolites according to the invention have rather a booster action. This is also confirmed by the fact that without a UV filter, zeolites of the invention have no action as a solar filter.

As will be apparent to a person skilled in the art, the composition of the invention can be included in diverse items and products such as: leave-on skin lotions and creams, shampoos, hair conditioners, shower gels, toilette bars, antiperspirants, deodorants, shave creams, lipsticks, lip-balms, stick formulations, foundations, sunscreen lotions/creams and the like. It is noted that sunscreen agents are active only when exposed to UV radiations. In cases where no prevention against UV radiations/sunburn is needed, for example when the composition is used as a shower gel, applying the composition to the skin cannot be considered as a therapeutic treatment and the composition is mostly used as a cosmetic rather than a medicament.

Therefore, the present invention also refers to non-therapeutic uses, e.g., cosmetic uses, of the composition, according to any one of the embodiments disclosed herein.

Other aspects and advantages of the invention will be apparent from the experimental section below.

## EXAMPLES

Hereinafter, the invention will be explained in more detail with reference to the following non-limiting examples. Modifications or variations of the embodiments here exemplified, obvious to an expert in the art, are encompassed by the appended claims.

The comparative and inventive sunscreen compositions were prepared according to the following methods incorporating the ingredients as listed in Table 1 and 2, and the SPF compared.

Manufacturing process for Oil in Water cream (B1, S1, S2, C1):

1. Weight components of phase A and heat up to 75-80°C, under stirring.

2. When the temperature is reached, add phase B and homogenize with UltraTurrax for 5 minutes at least 3500 rpm.

3. Weight components of phase C and heat up to 75-80°C, under stirring.

4. When both emulsifier and the UV filter are completely dissolved, add phase D and continue to mix until the SPF booster is fully incorporated in phase C.

5. When phases A+B and C+D are uniform and at their designated temperatures, add phases C+D to phases A+B homogenize with UltraTurrax at 4000 rpm for 10 minutes.

6. Cool down to room temperature under gentle stirring, then add one by one ingredients of Phase E.

7. Adjust the pH to 5.5-6.5 with citric acid if necessary

Table 1: Inventive and comparative example in oil-in water formulations

| Phase | INCI | % W/W B1 | %W/W S1 | %W/W S2 | %W/W C1 |
|---|---|---|---|---|---|
| A | Aqua | 65.2 | 63.2 | 63.2 | 63.2 |
| | Glycerin | 5 | 5 | 5 | 5 |
| | Tetrasodium Glutamate Diacetate | 0.1 | 0.1 | 0.1 | 0.1 |
| B | Xanthan Gum | 0.5 | 0.5 | 0.5 | 0.5 |
| C | Cetearyl Alchol | 3 | 3 | 3 | 3 |

(continued)

| Phase | INCI | % W/W B1 | %W/W S1 | %W/W S2 | %W/W C1 |
|---|---|---|---|---|---|
| | Glyceryl Stearate | 4 | 4 | 4 | 4 |
| | Caprylic/Capryc Triglyceride | 3.8 | 3.8 | 3.8 | 3.8 |
| | C12-C15 Alkyl benzoate | 4.8 | 4.8 | 4.8 | 4.8 |
| | Bis-Ethylhexyoxyphenol MethoxyPhenyl Triazine | 2 | 2 | 2 | 2 |
| | Butyl Methoxydibenzoylmethane | 2.5 | 2.5 | 2.5 | 2.5 |
| | Ethylhexyl methoxycinnamate | 7 | 7 | 7 | 7 |
| D | SPF booster ZSM-5/NH4+ zeolite functionalized with thiethoxycaprylyl silane | - | 2 | - | - |
| | SPF booster ZSM-5/NH4+ zeolite functionalized with thiethoxycaprylyl silane | - | - | 5 | - |
| | SPF booster ZSM-5/NH4+ zeolite | - | - | - | 2 |
| E | Phenylpropanol and propanediol and caprylyl glycol and Tocopherol | 1 | 1 | 1 | 1 |
| | Tocopheryl acetate | 0.4 | 0.4 | 0.4 | 0.4 |
| | Fragrance | 0.5 | 0.5 | 0.5 | 0.5 |
| F | Citric Acid, solution in water 30% | 0.2 | 0.2 | 0.2 | 0.2 |

Manufacturing process stick formulation (B2, S3):

1. Combine and mix together the reagents of phase A and heat up at 80-85°C, until well melted and homogenous.
2. Separately, blend together all the reagents of phase B and heat up at 80-85°C, until well melted and homogenous.
3. Add phase B to phase A and stir until homogenous.
4. Take it off the heat and mix with prior-blended phase C.
5. Pour off while still molten.

Table 2: Inventive and comparative example in stick formulations

| Phase | INCI | %W/W B2 | % W/W S3 |
|---|---|---|---|
| A | Caprylic capric triglicerides | 4.11 | 3.99 |
| | Isoamyl laurate | 15.75 | 15.30 |
| | C12-C15 Alkyl benzoate | 9.75 | 9.45 |
| | Caprylic/Capric Triglyceride (and) Polyurethane-79 | 50.71 | 49.20 |
| | SPF booster ZSM-5/$NH_4^+$ functionalized with thiethoxycaprylyl silane | 0 | 3.00 |
| | | | |
| B | Diethylamino hydroxybenzoyl hexyl benzoate | 6.70 | 6.50 |
| | Bis-Ethylhexyloxyphenol Methoxyphenyl Triazine | 9.78 | 9.49 |
| C | Zea Mays Germ Oil, Daucus carota sativa Root Extract | 1.37 | 1.33 |
| | Methyl Glucose Dioleate | 1.22 | 1.18 |
| | Parfum | 0.61 | 0.59 |

Manufacturing process stick formulation (B3, S4, S5, C2, C3):

1. Combine and mix together the reagents of phase A and heat up at 80-85°C, until well melted and homogenous.
2. Separately, blend together all the reagents of phase B and heat up at 80-85°C, until well melted and homogenous.

3. Add phase B to phase A and stir until homogenous.
4. Take it off the heat and mix with prior-blended phase C.
5. Pour off while still molten.

Table 3: Inventive and comparative example in stick formulations

| Phase | INCI | % W/W B3 | % W/W S4 | % W/W S5 | % W/W C2 | % W/W C3 |
|---|---|---|---|---|---|---|
| A | Caprylic capric triglicerides | 4.11 | 3.99 | 3.99 | 3.99 | 3.99 |
| | Isoamyl laurate | 15.75 | 15.30 | 15.30 | 15.30 | 15.30 |
| | C12-C15 Alkyl benzoate | 9.75 | 9.45 | 9.45 | 9.45 | 9.45 |
| | Caprylic/Capric Triglyceride (and) Polyurethane-79 | 50.71 | 49.20 | 49.20 | 49.20 | 49.20 |
| | SPF booster BETA 23.8/$NH_4^+$ functionalized with silane | 0 | 3.00 | 0 | 0 | 0 |
| | SPF booster BETA 307/$H^+$ functionalized with silane | 0 | 0 | 3.00 | 0 | 0 |
| | SPF booster BETA 23.8/$NH_4^+$ | 0 | 0 | 0 | 3.00 | 0 |
| | SPF booster BETA 307/$H^+$ | 0 | 0 | 0 | 0 | 3.00 |
| B | Diethylamino hydroxybenzoyl hexyl benzoate | 6.70 | 6.50 | 6.50 | 6.50 | 6.50 |
| | Bis-Ethylhexyloxyphenol Methoxyphenyl Triazine | 9.78 | 9.49 | 9.49 | 9.49 | 9.49 |
| C | Zea Mays Germ Oil, Daucus carota sativa Root Extract | 1.37 | 1.33 | 1.33 | 1.33 | 1.33 |
| | Methyl Glucose Dioleate | 1.22 | 1.18 | 1.18 | 1.18 | 1.18 |
| | Parfum | 0.61 | 0.59 | 0.59 | 0.59 | 0.59 |

SPF In vitro evaluation

It has been developed an internal *full in-vitro* method to calculate the protection factor (SPF $_{in\ vitro}$) of a sunprotection product against erythema-inducing radiation calculated with a calibration based on a set of sunprotection standards.

The method is formulated according to the principles recommended (Protocol named "In vitro SPF Double Plate method) by the European Cosmetics and Perfumery association (former Colipa, then Cosmetics Europe) in 2011 and internally modified. By consequence, the calculated protection factor is an internal value used to compare the samples and may be not directly comparable with fully regulated in vivo tests.

The test is based on the assessment of UV-transmittance through a thin film of sunscreen sample spread on a roughened PMMA (Poly(methyl-methacrylate)) substrate. In the inspiring procedure, exposure to a radiation from an UV exposure source is required. UV exposure is not used in this method; each set of sunscreen transmission data is mathematically corrected so that the in vitro SPF data yield the same SPF values provided by several certified SPF standards.

A Perkin-Elmer LAMBDA 1050+ UV/Vis/NIR spectrophotometer equipped with a 150 mm Perkin-Elmer LAMBDA integrating sphere is used to measure the absorbance A properties of the sunscreen on the test plates and absorbance values are used to calculate SPF through the following integral formula:

$$SPF_{in\ vitro} = \frac{\int_{290\ nm}^{400\ nm} E(\lambda) \times I(\lambda) d\lambda}{\int_{290\ nm}^{400\ nm} E(\lambda) \times I(\lambda) \times 10^{-C \times A(\lambda)} d\lambda}$$

Where:

E: tabled CIE-1987 erythema action spectrum;
I: tabled Midday mid-summer global irradiance at 40°N;
A: measured absorbance;

C: correction coefficient

The UV spectrophotometer wavelength range shall span the primary waveband of 290 to 400 nm, covering both the ranges 290-320 nm (UV-B) and 320-400 nm (UV-A) required in SPF calculation.

A correction coefficient such that $SPF_{invitro} = SPF_{i,standard}$ , i=P1, P2, P3 (certified standards in different ranges of sunprotection, see table herebelow for certified values) is used in the calculation.

| Mean and Acceptance limits of SPF reference formulations - Compliance ISO 24444:2019 | | | |
|---|---|---|---|
| formulation | mean | Lower Limit | Upper Limit |
| P2 | 16.,1 | 13.7 | 18.5 |
| P5 | 30.6 | 23.7 | 37.4 |
| P8 | 63.1 | 43.9 | 82.3 |

The UV spectrophotometer wavelength range shall span the primary waveband of 290 to 400 nm, covering both the ranges 290-320 nm (UV-B) and 320-400 nm (UV-A).

In the following table it is reported a mean SPF boosting value (%), calculated as:

$$mean\ SPF\ boosting\ (\%) = \frac{[(SPF_B - SPF_P) - (SPF_F - SPF_P)]}{SPF_F - SPF_P} \times 100$$

Where:

SPF$_B$: measured SPF of sample formulation with UV filter plus UV booster
SPF$_F$: measured SPF of sample formulation with UV filter
SPF$_P$: measured SPF of placebo formulation (without UV filter and UV booster)

The in-vitro results for both oil-in-water and stick formulations are reported in the following table.

Table 4

| Ref. | Formulation | Ingredient type as SPF booster | SPF value ± StDev | % SPF boosting |
|---|---|---|---|---|
| B1 | Cream | no | 13.5 ± 1.2 | Negligible |
| S1 | Cream | ZSM-5/NH$_4$$^+$ functionalized with silane | 30.5 ± 0.7 | 126 |
| S2 | Cream | ZSM-5/NH$_4$$^+$ functionalized with silane | 57.5 ± 9.3 | 326 |
| C1 | Cream | ZSM-5/NH$_4$$^+$ | 23.1 ± 9.9 | 71 |
| B2 | Stick | no | 87 ± 9 | Negligible |
| S3 | Stick | ZSM-5/NH$_4$$^+$ functionalized with silane | 125 ± 19 | 44 |
| B3 | Stick | no | 26.4 ± 3.9 | Negligible |
| S4 | Stick | SPF booster BETA 23.8/NH4+ functionalized with silane | 135.6±37.4 | 413 |
| S5 | Stick | SPF booster BETA 307/H+ functionalized with silane | 86.4±22.9 | 227 |
| C2 | Stick | SPF booster BETA 23.8/NH4+ | 60.1±9.8 | 128 |
| C3 | Stick | SPF booster BETA 307/H+ | 52.6±11.8 | 99 |

Table 4, clearly shows that the presence of an SPF booster based on zeolites according to the present invention, and specifically functionalized with silane moieties, contribute to significantly boost the UVB performances.

Indeed, when a ZSM-5 type zeolite functionalized with a thiethoxycaprylyl silane is employed as SPF booster in both stick and cream formulations (see S1-S3), the UVB performance has an increase higher than 40% with respect to the reference compositions which do not contain any SPF booster systems. Also, when a BETA type zeolite functionalized with a thiethoxycaprylyl silane is employed as SPF booster in a stick formulation (see S4-S5), the UVB performance has an increase higher than 200% with respect to the reference compositions (B3) which do not contain any SPF booster systems.

On the contrary, as disclosed in counterexample C1-C3, the same zeolites particles which do not have the silane functionalization do not reach the same boosting effect.

## Claims

1. A sunscreen composition comprising:

   a) at least one UV filter; and
   b) zeolites as SPF boosters,

   wherein said zeolites are selected in a group consisting of Beta type, ZSM-5 type (MFI), Linde type (LTA), Mordenite type (MOR) and Faujasite type (FAU) functionalized with a silane moiety selected among thiethoxycaprylyl silane Stearyl Triethoxysilane, Trihydroxymethylsilane or Methoxypolyethyleneoxypropyltrymetoxysilane.

2. A composition according to claim 1, wherein the zeolites comprise ammonium or hydrogen as counter-ion at an ion exchange site of the framework structure.

3. A composition according to claim 1 or 2, wherein the particle size distribution is comprised between 0.1 and 15 $\mu$m, preferably between 0.2 and 5.0 $\mu$m.

4. A composition according to any of the previous claims, wherein the amount of zeolite is comprised between 0.1 and 5.0% with respect to the total weight of the composition.

5. A composition according to any of the previous claims, wherein the amount of silane is comprised between 0.2 and 10.0 % preferably between 2.0 and 6.0 % with respect the total weight of the zeolite.

6. A composition according to any of the previous claims, wherein the UV filter is an organic UV filter in an amount comprised between 1 and 15% wt with respect to the total amount of the composition.

7. A composition according claim 6, wherein the UV filter is an organic molecule selected in a group consisting of Octocrylene, Ethylhexyl Salicylate, Homosalate, Butyl Methoxydibenzoylmethane, Diethylamino hydroxybenzoyl hexyl benzoate, Bis-Ethylhexyloxyphenol Methoxyphenyl Triazine, Ethylhexyl Triazone, Avobenzone, Diethylhexyl Butamido Triazone, Ethylhexyl Methoxycinnamate, Phenylbenzimidazole sulfonic acid, Tris-biphenyl triazine, Tris-biphenyltrazine (nano), Oxybenzone, Octinoxate, Octyltriazone, Octisalate, Padimate O, Bis-piperazine HAA299, Bis-piperazine HAA299 (nano), (2-Ethoxyethyl (2Z)-2-cyano-2-[3-(3-methoxypropylamino) cyclohex-2-en-1-ylidene]acetate), Sulisobenzone, Drometrizole trisiloxane, Methylene bis-benzotriazolyl tetramethylbutylphenol, Benzylidene camphor sulfonic acid, 4-Methylbenzylidene Camphor, Polysilicone-15, Sulisobenzone sodium, Aminobenzoic acid, PEG-25 PABA, Methylene Bis-Benzotriazolyl Tetramethylbutylphenol, Methylene Bis-Benzotriazolyl Tetramethylbutylphenol nano, Menthyl anthranilate, Isopentyl-4-methoxycinnamate, terephthalylidene dicamphor sulfonic acid, Dioxibenzone, Cinoxate, camphor benzalkonium sulfate and mixtures thereof.

8. A composition according to claim 1 to 6, wherein the UV filter is an inorganic compound in an amount comprised between 1 and 25% wt.

9. A composition according to claim 8, wherein the UV filter is an inorganic compound selected in a group consisting of Zinc Oxide, Anatase titanium dioxide, Rutile, titanium dioxide and mixtures thereof considering all size range both nano-, sub- and micro-metric.

10. A composition according to any of the previous claims, wherein the UV filters are a mixture of organic UV filter and inorganic UV filters.

11. A composition according to any of the previous claims, wherein the zeolites provide an SPF-boosting action to the sunscreen composition of at least 20%.

12. A composition according to any of the previous claims, further comprising at least one of a cosmetically acceptable emollient, carrier fluid, pigment, humectant, vitamin, antioxidant, emulsifier, co-emulsifier, hydrophilic or hydrophobic thickeners, wax, butter, film former, silicone, surfactant, active agent, extract, fragrance, preservative, pH-adjusting agent, suspending agent, chelating agent.

13. A composition according to any of the previous claims formulated as a stick.

14. A composition according to any of the previous claims, wherein the composition is a homogenous oil-in-water emulsion.

15. A composition according to any one of claims 1 to 14 for use as a sunscreen agent, particularly for use in a method for protecting a keratinous surface from UV radiation comprising contacting the keratinous surface with said sunscreen composition.

**Patentansprüche**

1. Sonnenschutzzusammensetzung, umfassend:

   a) mindestens einen UV-Filter; und
   b) Zeolithe als LSF-Verstärker,

   wobei die Zeolithe ausgewählt sind aus einer Gruppe, bestehend aus Beta-Typ, ZSM-5-Typ (MFI), Linde-Typ (LTA), Mordenit-Typ (MOR) und FaujasitTyp (FAU), funktionalisiert mit einem Silanrest ausgewählt unter Triethoxycaprylylsilan, Stearyltriethoxysilan, Trihydroxymethylsilan oder Methoxypolyethyleneoxypropyltrymetoxysilan.

2. Zusammensetzung nach Anspruch 1, wobei die Zeolithe Ammonium oder Wasserstoff als Gegenion an einer Ionenaustauschstelle der Rahmenstruktur umfassen.

3. Zusammensetzung nach Anspruch 1 oder 2, wobei die Teilchengrößenverteilung zwischen 0,1 und 15 $\mu$m liegt, vorzugsweise zwischen 0,2 und 5,0 $\mu$m.

4. Zusammensetzung nach einem der vorstehenden Ansprüche, wobei die Menge von Zeolith zwischen 0,1 und 5,0 %, bezogen auf das Gesamtgewicht der Zusammensetzung, liegt.

5. Zusammensetzung nach einem der vorstehenden Ansprüche, wobei die Menge von Silan zwischen 0,2 und 10,0 %, vorzugsweise zwischen 2,0 und 6,0 %, bezogen auf das Gesamtgewicht des Zeoliths, liegt.

6. Zusammensetzung nach einem der vorstehenden Ansprüche, wobei der UV-Filter ein organischer UV-Filter in einer Menge zwischen 1 und 15 Gew.-%, bezogen auf die Gesamtmenge der Zusammensetzung, ist.

7. Zusammensetzung nach Anspruch 6, wobei der UV-Filter ein organisches Molekül ist, ausgewählt aus einer Gruppe, bestehend aus Octocrylen, Ethylhexylsalicylat, Homosalat, Butylmethoxydibenzoylmethan, Diethylaminohydroxybenzoylhexylbenzoat, Bis-Ethylhexyloxyphenolmethoxyphenyltriazin, Ethylhexyltriazon, Avobenzon, Diethylhexylbutamidotriazon, Ethylhexylmethoxycinnamat, Phenylbenzimidazolsulfonsäure, Tris-Biphenyltriazin, Tris-Biphenyltriazin (nano), Oxybenzon, Octinoxat, Octyltriazon, Octisalat, Padimat O, Bis-Piperazin HAA299, Bis-Piperazin HAA299 (nano), (2-Ethoxyethyl-(2Z)-2-cyano-2-[3-(3-methoxypropylamino)cyclohex-2-en-1-yliden]acetat), Sulisobenzon, Drometrizoltrisiloxan, Methylenbis-benzotriazolyltetramethylbutylphenol, Benzylidenkampfersulfonsäure, 4-Methylbenzylidenkampfer, Polysilikon-15, Sulisobenzonnatrium, Aminobenzoesäure, PEG-25 PABA, Methylenbis-Benzotriazolyltetramethylbutylphenol, Methylenbis-Benzotriazolyltetramethylbutylphenol nano, Menthylanthranilat, Isopentyl-4-methoxycinnamat, Terephthalylidendikampfersulfonsäure, Dioxibenzon, Cinoxat, Kampferbenzalkoniumsulfat und Mischungen davon.

8. Zusammensetzung nach Anspruch 1 bis 6, wobei der UV-Filter eine anorganische Verbindung in einer Menge zwischen 1 und 25 Gew.-% ist.

9. Zusammensetzung nach Anspruch 8, wobei der UV-Filter eine anorganische Verbindung ist, ausgewählt aus einer Gruppe, bestehend aus Zinkoxid, Anatastitandioxid, Rutil, Titandioxid und Mischungen davon unter Berücksichtigung aller Größenbereiche sowohl nano-, sub- als auch mikrometrisch.

10. Zusammensetzung nach einem der vorstehenden Ansprüche, wobei die UV-Filter eine Mischung aus organischen UV-Filtern und anorganischen UV-Filtern sind.

11. Zusammensetzung nach einem der vorstehenden Ansprüche, wobei die Zeolithe eine LSF-verstärkende Wirkung auf die Sonnenschutzzusammensetzung von mindestens 20 % bereitstellen.

12. Zusammensetzung nach einem der vorstehenden Ansprüche, ferner umfassend mindestens eines von einem kosmetisch verträglichen Weichmacher, Trägerfluid, Pigment, Feuchthaltemittel, Vitamin, Antioxidans, Emulgator, CoEmulgator, hydrophilen oder hydrophoben Verdickungsmitteln, Wachs, Butter, Filmbildner, Silikon, Tensid, Wirkstoff, Extrakt, Duftstoff, Konservierungsmittel, pH-Einstellmittel, Suspensionsmittel, Chelatbildner.

13. Zusammensetzung nach einem der vorstehenden Ansprüche, formuliert als ein Stift.

14. Zusammensetzung nach einem der vorstehenden Ansprüche, wobei die Zusammensetzung eine homogene Öl-in-Wasser-Emulsion ist.

15. Zusammensetzung nach einem der Ansprüche 1 bis 14 zur Verwendung als ein Sonnenschutzmittel, insbesondere zur Verwendung in einem Verfahren zum Schutz einer keratinösen Oberfläche vor UV-Strahlung, umfassend ein Inberührungbringen der keratinösen Oberfläche mit der Sonnenschutzzusammensetzung.

## Revendications

1. Composition de protection solaire comprenant :

    a) au moins un filtre UV ; et
    b) des zéolithes en tant qu'agents amplificateurs de facteur de protection solaire,

    dans laquelle lesdites zéolithes sont choisies dans un groupe constitué par des zéolithes de type bêta, type ZSM-5 (MFI), type Linde (LTA), type mordénite (MOR) et type faujasite (FAU) fonctionnalisées par un groupement silane choisi parmi thiéthoxy caprylyl silane, stéaryl triéthoxysilane, trihydroxyméthylsilane ou méthoxypolyéthylèneoxypropyltrymétoxysilane.

2. Composition selon la revendication 1, dans laquelle les zéolithes comprennent de l'ammonium ou de l'hydrogène en tant que contre-ion au niveau d'un site d'échange d'ions de la structure d'ossature.

3. Composition selon la revendication 1 ou 2, dans laquelle la distribution granulométrique est comprise entre 0,1 et 15 μm, de préférence entre 0,2 et 5,0 μm.

4. Composition selon l'une quelconque des revendications précédentes, dans laquelle la quantité de zéolithe est comprise entre 0,1 et 5,0 % par rapport au poids total de la composition.

5. Composition selon l'une quelconque des revendications précédentes, dans laquelle la quantité de silane est comprise entre 0,2 et 10,0 %, de préférence entre 2,0 et 6,0 % par rapport au poids total de la zéolithe.

6. Composition selon l'une quelconque des revendications précédentes, dans laquelle le filtre UV est un filtre UV organique en une quantité comprise entre 1 et 15 % en poids par rapport à la quantité totale de la composition.

7. Composition selon la revendication 6, dans laquelle le filtre UV est une molécule organique choisie dans un groupe constitué par l'octocrylène, salicylate d'éthylhexyle, homosalate, méthoxydibenzoylméthane de butyle, diéthylamino hydroxybenzoyl hexyl benzoate, bis-éthylhexyloxyphénol méthoxyphényl triazine, éthylhexyl triazone, avobenzone, diéthylhexyl butamido triazone, éthylhexyl méthoxycinnamate, acide phénylbenzimidazole sulfonique, tris-biphényltriazine, tris-biphényl trazine (nano), oxybenzone, octinoxate, octyltriazone, octisalate, padimate O, bis-pipérazine HAA299, bis-pipérazine HAA299 (nano), (2-éthoxyéthyl (2Z)-2-cyano-2-[3-(3-méthoxypropylamino) cyclohex-2-en-1-ylidène]acétate), sulisobenzone, drométrizole trisiloxane, méthylène bis-benzotriazolyl tétraméthylbutylphénol, acide benzylidène camphre sulfonique, 4-méthylbenzylidène camphre, polysilicone-15, sulisobenzone sodique, acide aminobenzoïque, PEG-25 PABA, méthylène bis-benzotriazolyl tétraméthylbutylphénol, méthylène bis-benzotriazolyl tétraméthylbutylphénol nano, anthranilate de menthyle, isopentyl-4-méthoxycinnamate, acide téréphtalidène dicamphre sulfonique, dioxibenzone, cinoxate, camphre benzalkonium sulfate et des mélanges de ceux-ci.

8. Composition selon la revendication 1 à 6, dans laquelle le filtre UV est un composé inorganique en une quantité comprise entre 1 et 25 % en poids.

9. Composition selon la revendication 8, dans laquelle le filtre UV est un composé inorganique choisi dans un groupe constitué par l'oxyde de zinc, dioxyde de titane anatase, rutile, dioxyde de titane et des mélanges de ceux-ci en tenant compte de toutes les plages de tailles à la fois nanométriques, submétriques et micrométriques.

10. Composition selon l'une quelconque des revendications précédentes, dans laquelle les filtres UV sont un mélange de filtres UV organiques et de filtres UV inorganiques.

11. Composition selon l'une quelconque des revendications précédentes, dans laquelle les zéolithes confèrent une action d'amplification de facteur de protection solaire d'au moins 20 % à la composition de protection solaire.

12. Composition selon l'une quelconque des revendications précédentes, comprenant en outre au moins l'un parmi un émollient cosmétiquement acceptable, fluide porteur, pigment, humectant, vitamine, antioxydant, émulsifiant, co-émulsifiant, épaississants hydrophiles ou hydrophobes, cire, beurre, agent filmogène, silicone, tensioactif, agent actif, extrait, parfum, conservateur, agent d'ajustement de pH, agent de suspension, agent chélatant.

13. Composition selon l'une quelconque des revendications précédentes, formulée sous forme de bâtonnet.

14. Composition selon l'une quelconque des revendications précédentes, dans laquelle la composition est une émulsion huile-dans-eau homogène.

15. Composition selon l'une quelconque des revendications 1 à 14, destinée à être utilisée en tant qu'agent de protection solaire, en particulier destinée à être utilisée dans un procédé pour la protection d'une surface kératinique contre un rayonnement UV comprenant la mise en contact de la surface kératinique avec ladite composition de protection solaire.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017112982 A
- WO 2021102873 A
- US 9265715 B
- US 2005276761 A
- US 20210330571 A
- WO 2022081942 A

**Non-patent literature cited in the description**

- In vitro SPF Double Plate method. European Cosmetics and Perfumery association, 2011